(19) Europäisches Patentamt
European Patent Office
Office européen des brevets

(11) Veröffentlichungsnummer: **0 266 711**
**A1**

(12) # EUROPÄISCHE PATENTANMELDUNG

(21) Anmeldenummer: 87116036.2

(22) Anmeldetag: 31.10.87

(51) Int. Cl.[4]: C07D 213/73, A61K 31/44

(30) Priorität: 06.11.86 DE 3637829

(43) Veröffentlichungstag der Anmeldung: 11.05.88 Patentblatt 88/19

(84) Benannte Vertragsstaaten: AT BE CH DE ES FR GB GR IT LI LU NL SE

(71) Anmelder: ASTA Pharma AG
Daimlerstrasse 25
D-6000 Frankfurt am Main 1(DE)

(72) Erfinder: Der weitere Erfinder hat auf seine Nennung verzichtet

(54) **Neue 2,6-Diamono-3-halogenobenzyl-pyridine und Verfahren zu ihrer Herstellung sowie ihre Verwendung in Pharmazeutika.**

(57) Die Erfindung betrifft neue 2,6-Diamino-3-halogenobenzylpyridine der Formel I

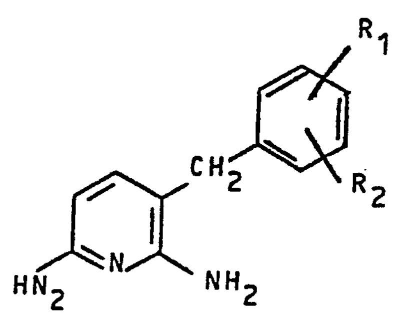

wobei $R_1$ Fluor und $R_2$ Wasserstoff oder Chlor bedeutet sowie deren physiologisch verträgliche Säureadditionssalze, Verfahren zu ihrer Herstellung und ihre Verwendung in Pharmazeutika.

EP 0 266 711 A1

## Neue 2,6-Diamino-3-halogenobenzylpyridine und Verfahren zu ihrer Herstellung sowie ihre Verwendung in Pharmazeutika

Die Erfindung betrifft neue 2,6-Diamino-3-halogenobenzyl-pyridine der Formel I

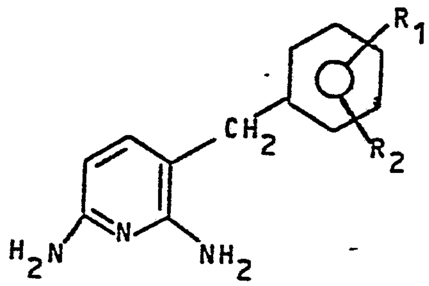

I

wobei $R_1$ Fluor und $R_2$ Wasserstoff oder Chlor bedeutet sowie deren physiologisch verträgliche Säureadditionssalze, Verfahren zur ihrer Herstellung und ihre Verwendung in Pharmazeutika.

In den deutschen Patentschriften DE PS 19 33 504 und DE PS 19 08 078 wird die baktericide Wirkung von 2-Alkylamino-6-aminopyridinen beschrieben. Als weitere pharmakologische Wirkung von substituierten 2,6-Diaminopyridinen ist nach der deutschen Offenlegungsschrift DOS 25 14 558.8 bekannt, diese zur Bekämpfung von Fettsucht und als Antidiabetika einzusetzen. Es wurde nun gefunden, daß die in 2,6-Stellung unsubstituierten 2,6-Diamino-3-halogenobenzylpyridine der Formel I analgetische und antipyretische Wirkungen zeigen.

Die neuen Verbindungen können beispielsweise hergestellt werden, indem man 2,6-Diaminopyridin mit einem Arylalkylhalogenid der Formel II

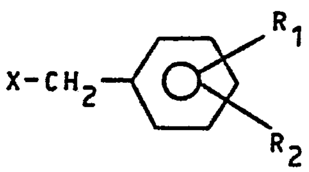

II

wobei X Halogen und $R_1$ Fluor und $R_2$ Wasserstoff oder Chlor bedeutet, umsetzt.

Nach der allgemeinen Methode wird hierbei ein äquimolares Gemisch 2,6-Diaminopyridin und Arylalkylhalogenid der Formel II unter Rühren langsam bis zur beginnenden Schmelze auf ca. 95°C bis 110°C erhitzt. Die Temperatur der Schmelze steigt sodann exotherm ohne äußere Wärmezufuhr auf ca. 200°C bis 250°C an und fällt nach beendeter Reaktion (ca. 20 Minuten) wieder ab. Der Verlauf der exothermen Reaktion kann gegebenenfalls durch Kühlung mit Eiswasser reguliert werden. Sodann wird die Schmelze ca. 2 Stunden bei 130°C gerührt und das Reaktionsgemisch nach Abkühlung auf 25°C in einem Gemisch von 400 ml Methylenchlorid und ca. 40 ml konzentriertem Ammoniak gelöst. Man extrahiert mit 2 × 150 ml Wasser, trennt die organische Phase ab, trocknet sie über wasserfreiem Natriumsulfat und engt sodann im Vakuum ein. Der verbleibende Rückstand wird säulenchromatographisch getrennt (geduran Si 60 Fa. Merck) Laufmittel: Methylenchlorid/Methanol beziehungsweise Ethanol, und die Reaktionsprodukte werden aufgrund ihrer unterschiedlichen Laufgeschwindigkeiten isoliert.

Die rein erhaltenen Verbindungen liegen als Basen vor und können gegebenenfalls durch Umsetzung mit Säuren in therapeutisch verwendbare Salze überführt werden.

Als solche Säuren seien beispielsweise genannt: Halogenwasserstoffsäuren, Schwefelsäure, Phosphorsäuren, Salpetersäure, Perchlorsäure, organische Mono-, Di-oder Tricarbonsäuren der aliphatischen, alicyclischen, aromatischen oder heterocyclischen Reihe sowie Sulfonsäuren. Beispiele hierfür sind: Ameisen-, Essig-, Propion-, Bernstein-, Glykol-, Milch-, Äpfel-, Wein-, Zitronen-, Ascorbin-, Malein-, Fumar-, Hydroxymalein-, Glucon-oder Brenztraubensäure; Phenylessig-, Benzoe-, p-Aminosalicylsäure, Embonsäure, Methansulfon-, Ethansulfon-, Hydroxyethansulfon-, Ethylensulfonsäure; Halogenbenzolsulfon-, Toluolsulfon-, Naphthalinsulfonsäure oder Sulfanilsäure oder auch 8-Chlor-theophyllin.

Die gleiche Reaktion läßt sich auch vorteilhafter durchführen, indem man eine molare Menge 2,6-Diaminopyridin vorlegt, durch langsames Erwärmen zur Schmelze bringt und nun bei 100°C bis 130°C vorzugsweise bei 115°C bis 120°C eine äquimolare Menge eines flüssigen Arylalkylhalogenids zutropft. Die Temperatur steigt sodann exotherm auf etwa 140°C bis 160°C an. Es wird noch ca. 4 Stunden bei 130°C bis 150°C erhitzt, danach abgekühlt und das sirupöse Gemisch wird nun in 400 ml Methylenchlorid und ca. 40 ml konzentriertem Ammoniak gelöst.

Die Aufarbeitung und säulenchromatographische Trennung der Reaktionsprodukte erfolgt in der obengenannten Weise. Ein weiteres Verfahren zur Herstellung der Verbindungen nach Formel I bietet beispielsweise die Reaktion von 2,6 Diaminopyridin mit einem Arylalkylamin der Formel III

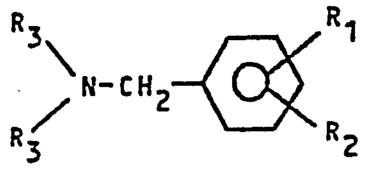

III

wobei $R_3$ $C_1$-$C_4$-Alkyl und $R_1$ Fluor und $R_2$ Wasserstoff oder Chlor bedeutet in Gegenwart von Alkalialkoholat. Als Alkalialkoholate kommen die Na,K-salze von niederen aliphatischen $C_1$-$C_4$-Alkoholen in Betracht.

Man geht hierbei bevorzugt von einem mindestens 4-fach molaren Überschuß des 2,6-Diaminopyridins aus, um die ebenfalls in 3,5-Position des Pyridinrings disubstituierten und die N-benzylierten Nebenprodukte des 2,6-Diaminopyridins gering zu halten. Die Reaktion erfolgt in einem inerten organischen Lösungsmittel bei Temperaturen zwischen 100°C und 150°C in einer Schutzgasatmosphäre wie zum Beispiel $N_2$ nach der Methode von B.S.Rauckman und B.Roth J.Med.Chem.23 384, 1980. Als Lösungsmittel kommen beispielsweise niedere $C_1$-$C_4$-Alkohole, niedere Diole, niedere $C_1$-$C_4$-Ether, niedere cyclische Ether wie beispielsweise Methoxyethanol, Dioxan, Ethylenglycol u.a. in Betracht. Die Aufarbeitung und säulenchromatographische Trennung der Reaktionsprodukte erfolgt ebenfalls in der obengenannten Weise.

In einem weiteren Verfahren wird eine Lösung von 2,6-Diaminopyridin und der einfach oder doppelt molaren Menge des Arylalkylhalogenids in einem polaren inerten organischen Lösungsmittel auf 90 - 110°C beziehungsweise bei Siedetemperatur des Lösungsmittels 8 Stunden unter Stickstoff erhitzt, nach Abkühlen des Reaktionsgemischs das Lösungsmittel abdestilliert und der verbleibende Rückstand in der obengenannten Weise aufgearbeitet und säulenchromatographisch gereinigt.

Als Lösungsmittel kommen zum Beispiel in Betracht:

niedere aliphatische Ketone wie zum Beispiel Aceton, Methyläthylketon; halogenierte Kohlenwasserstoffe wie zum Beispiel Chloroform, Tetrachlorkohlenstoff, Chlorbenzol, Methylenchlorid; cyclische Ether wie zum Beispiel Tetrahydrofuran und Dioxan; niedere aliphatische nichtcyclische Ether (Diethylether, Diisopropylether); niedere aliphatische Alkohole (1-6 C-Atome), wie zum Beispiel Methanol, Äthanol, Isopropanol, Amylalkohol, Butanol, tert.-Butanol; $C_1$-$C_4$-Alkoxisubstituierte Alkohole (Methoxyethanol) Amide und N-alkylsubstituierte Amide von aliphatischen $C_1$-$C_4$-Carbonsäuren (Dimethylformamid, Dimethylacetamid); $C_1$-$C_6$-Dialkylsulfone (Dimethylsulfon, Tetramethylsulfon); $C_1$-$C_6$-Dialkylsulfoxide (Dimethylsulfoxid), Di-und mehrfunktonelle aliphatische Alkohole (Ethylenglycol), sowie weitere aprotische Mittel wie N-Methylpyrrolidon, Tetramethylharnstoff, Hexamethylphosphorsäuretriamid, Acetonitril, Propionitril.

Die einzelnen Alkylreste der oben angegebenen Lösungsmittel enthalten beispielsweise 1-6, insbesondere 1-4 Kohlenstoffatome. Auch Gemische dieser Mittel sowie Mischungen mit Wasser kommen als Reaktionsmedium in Frage.

Die nach diesem Verfahren hergestellten Verbindungen der Formel I sind bevorzugt im Phenylring in ortho- und/oder para-Stellung mit Halogen substituiert. Hierbei steht besonders bevorzugt für $R_1$ und $R_2$ Fluor und/oder Chlor. 2,6-Diamino-3-(4-fluoro)-benzylpyridin und deren physiologisch verträglichen Säureadditionssalze zeigen besonders günstige Eigenschaften.

Nach den vorstehenden Herstellungsmethoden wurden folgende Verbindungen der Formel I hergestellt

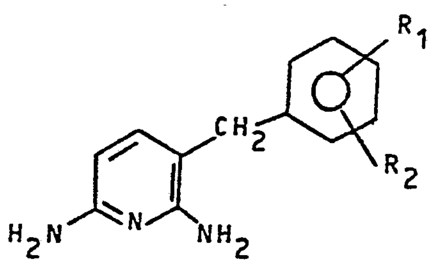

| | | | |
|---|---|---|---|
| D 17033 | $R_1$ = H | $R_2$ = p-Fluor | Fp: 123 - 24°C |
| D 17746 | $R_1$ = H | $R_2$ = o-Fluor | Fp: 115° - 17°C |
| D 17748 | $R_1$ = o-Fluor | $R_2$ = o-Chlor | Fp: 108 - 111°C |

Die Verbindungen der Formel I beziehungsweise ihre physiologisch verträglichen Säureadditionssalze zeigen im Tierversuch wertvolle therapeutische, insbesondere zentral-und peripher-analgetische sowie antipyretische Eigenschaften. Insbesondere zeichnet sich die Verbindung D 17033 gegenüber bekannten Substanzen, wie zum Beispiel den nichtsteroidalen Antiphlogistika mit analgetischer Komponente durch ein weitgehendes Fehlen von Nebenwirkungen wie zum Beispiel gastrointestinale Störungen und Magenulzera aus.

Die erfindungsgemäßen Wirkstoffe beziehungsweise ihre Salze können in die üblichen galenischen Anwendungsformen, wie Tabletten, Dragées, Lösungen, Emulsionen, Pulver, Kapseln oder Depotformen gebracht werden, wobei zu deren Herstellung die üblichen pharmazeutischen Hilfsstoffe sowie die üblichen Fertigungsmethoden herangezogen werden können. Entsprechende Tabletten können beispielsweise durch Mischen der Wirkstoffe mit bekannten Hilfsstoffen, beispielsweise inerten Verdünnungsmitteln wie Calciumcarbonat, Calciumphosphat oder Milchzucker, Sprengmitteln wie Maisstärke oder Alginsäure, Bindemitteln wie Stärke oder Gelatine, Schmiermitteln wie Magnesiumstearat oder Talk, und/oder Mitteln zur Erzielung eines Depoteffekts wie Carboxypolymethylen, Carboxymethylcellulose, Celluloseacetatphthalat, oder Polyvinylacetat erhalten werden.

Die Tabletten können auch aus mehreren Schichten bestehen. Entsprechend können Dragées durch Überziehen von analog den Tabletten hergestellten Kernen mit üblicherweise in Dragéeüberzügen verwendeten Mitteln, beispielsweise Kollidon oder Schellack, Gummi arabicum, Talk, Titandioxid oder Zucker, hergestellt werden. Zur Erzielung eines Depoteffekts oder zur Vermeidung von Inkompatibilitäten kann der Kern auch aus mehreren Schichten bestehen. Desgleichen kann auch die Dragéehülle zur Erzielung eines Depoteffekts aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombination können zusätzlich noch ein Süssungsmittel, wie Saccharin, Cyclamat, Glycerin oder Zucker, sowie ein geschmackverbesserndes Mittel, zum Beispiel Aromastoffe, wie Vanillin oder Orangenextrakt, enthalten.

Sie können außerdem Suspendierhilfsstoffe oder Dickungsmittel, wie Natriumcarboxymethyl-Cellulose, Netzmittel, beispielsweise Kondensationsprodukte von Fettalkoholen mit Äthylenoxid, oder Schutzstoffe, wie p-Hydroxybenzonate, enthalten.

Injektionslösungen werden in üblicher Weise, zum Beispiel unter Zusatz von Konservierungsmitteln, wie p-Hydroxybenzoaten, oder Stabilisatoren, wie Komplexonen, hergestellt und in Injektionsflaschen oder Ampullen abgefüllt.

Die Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen der dafür vorgesehenen Wirkstoffe beziehungsweise Wirkstoffkombinationen mit üblichen Trägermitteln, wie Neutralfetten oder Polyäthylenglykol beziehungsweise dessen Derivaten, herstellen.

Beispiel 1

2,6-Diamino-3(4-fluor)benzylpyridin (D 17033)

10,9 g (0,1M) 2,6-Diaminopyridin werden durch langsames Erwärmen zur Schmelze gebracht und bei 115°C - 120°C werden 14,45 g (0,1M) 4-Fluorbenzylchlorid hinzugetropft. Nachdem sich die Reaktionsmischung exotherm auf ca. 140°C - 160°C erwärmt hat, wird noch ca. 4 Stunden bei 130°C - 150°C erhitzt, danach wird das Reaktionsgefäß abgekühlt und das sirupöse Gemisch wird in 400 ml Methylenchlorid und ca. 40 ml konzentriertem Ammoniak gelöst. Die Reaktionslösung wird mit Wasser extrahiert und die organische Phase wird sodann abgetrennt und über wasserfreiem Natriumsulfat getrocknet. Das Lösungsmittel wird im Vakuum verdampft, und der verbleibende Rückstand wird säulenchromatographisch getrennt (Geduran Si 60 Fa. Merck AG Darmstadt Laufmittel: Methylenchlorid/Ethanol = 9:1 Vol.%). Man erhält 12,6 g 2,6-Diamino-3(4-fluor)benzyl-pyridin mit einem Schmelzpunkt: 123° - 124°C. (Ausbeute 58% d. Th.)
Dünnschichtchromatographie: $R_F$-Wert: 0,34
Laufmittel: Methylenchlorid/Ethanol/konz. Ammoniak = 95:4:1 Vol.%)

Nach dem Verdampfen des Lösungsmittels kann der verbleibende Rückstand auch durch fraktionierte Destillation im Hochvakuum gereinigt werden (Kp.: 190 - 210°C bei 0,4 m bar).

Beispiel 2:

2,6-Diamino-3(4-fluor)benzylpyridin-maleat-3,12 g 2,6-Diamino-3(4-fluor)benzylpyridin werden in 33 ml Methylenchlorid gelöst und mit einer Lösung von 1,66 g Maleinsäure in Ether fraktioniert gefällt. Das über Nacht kristallin anfallende Maleat saugt man ab, wäscht es mit eisgekühltem Methylenchlorid nach und suspendiert den Niederschlag in ca. 15 ml Ether. Filtration ergibt 3,6 g 2,6-Diamino-3-(4-fluor)benzylpyridin Maleat. Durch Umkristallisation aus heißem Ethanol erhält man 3 g dünnschichtchromatographisch (Dünnschichtchromatographie an Kieselgel-Fertigplatten 60 $F_{254}$ Fa. Merck AG; Laufmittel: Methylenchlorid/Ethanol 9 : 1, Entwicklung durch Fluoreszens im UV-Licht und Jod-Dampf, $R_F$ = 0,5) einheitliches 2,6-Diamino-3-(4-fluor)benzylpyridin Maleat. Fp.: 161 - 162°C.

Beispiel 3:

2,6-Diamino-3(4-fluor)benzylpyridin-hydrochlorid (D 19 506) 1,51 g 2,6-Diamino-3(4-fluor)benzylpyridin werden in 47 ml Methylenchlorid gelöst und die Lösung unter Argon-Schutzgas bei intensivem Rühren tropfenweise mit 1,31 ml 5,42 N isopropanolischer Salzsäure versetzt. Nach ca. 10 Minuten setzt Kristallisation des HCl-Salzes ein. Man rührt ca. 1,5 Stunden bei Raumtemperatur nach, saugt sodann unter Argon-Bespühlung ab, wäscht zweimal mit jeweils 2 ml Methylenchlorid nach und suspendiert unter Rühren die rohen Kristalle in 15 ml Ether. Nach 1,5 Stunden wird abgesaugt und bei 40°C im Vakuum getrocknet.
Ausbeute: 1,6g, Fp.: 164 - 167°C.

In gleicher Weise werden hergestellt:
2,6-Diamino-3(2-fluor)benzylpiridin (D 17 746)
Schmelzpunkt: 115-117°C Ausbeute 62 % d. Th.
Säulenchromatographie: Laufmittel Methylenchlorid/Ethanol = 9:1 Vol.%)
Dünnschichtchromatographie: $R_F$-Wert: 0,56
Laufmittel: Methylenchlorid/Ethanol/konz.
Ammoniak = 90:10:1 Vol.%

2,6-Diamino-3(2-fluor-6-chlor)benzylpyridin (D 17 748)
Schmelzpunkt: 108° - 111°C Ausbeute: 55% d.Th.
Säulenchromatographie: Laufmittel Methylenchlorid/Ethanol = 9:1 Vol.%
Dünnschichtchromatographie: $R_F$ -Wert: 0,31
Laufmittel Methylenchlorid/Ethanol/konz.
Ammoniak = 95:4:1 Vol.%

Beispiel 4

2,6-Diamino-3(4-fluor)benzylpyridin (D 17 033) Ein Gemisch von 34,9 g (0,32 M) 2,6-Diaminopyridin, 12,2 g (0,08 M) N,N-Dimethyl-N-(4-fluorbenzyl)amin und 0,65 g (0,012 M) Natriummethylat werden in 190 ml Ethylenglykol unter Stickstoff-Atmosphäre auf 120°C erhitzt. Nach einer Reaktionszeit von 2 1/2 Stunden wird auf Raumtemperatur abgekühlt, und in die Mischung werden 9 ml Eisessig hinzugefügt. Anschließend wird das Lösungsmittel im Vakuum entfernt, und der verbleibende Rückstand wird in Methanol aufgenommen und mit Tierkohle behandelt. Nach dem Abfiltrieren der Tierkohle wird der Rückstand säulenchromatographisch wie in Beispiel 1 gereinigt. Man erhält 8,5 g 2,6-Diamino-3(4-fluor)benzylpyridin (Ausbeute: 49% d.Th.)

**Ansprüche**

1. 2,6-Diamino-3-halogenobenzyl-pyridine der Formel I

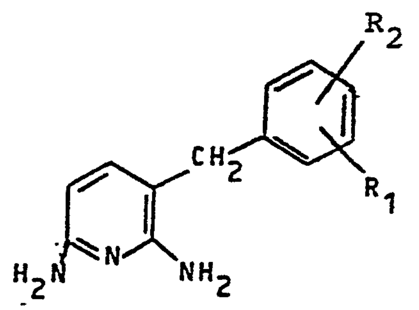

I

wobei $R_1$ Fluor und $R_2$ Wasserstoff oder Chlor bedeutet sowie deren physiologisch verträgliche Säureadditionssalze

2. Verfahren zur Herstellung von 2,6-Diamino-3-halogenobenzyl-pyridinen der Formel I und deren physiologisch verträgliche Säureadditionssalze

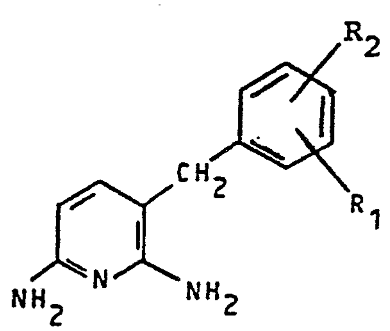

wobei $R_1$ Fluor und $R_2$ Wasserstoff oder Chlor bedeutet, dadurch gekennzeichnet, daß 2,6-Diaminopyridin mit einem Arylalkylhalogenid der Formel II

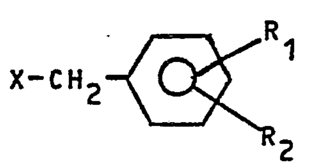

II

wobei X Halogen und $R_1$ Fluor und $R_2$ Wasserstoff oder Chlor bedeutet oder mit einem Arylalkylamin der Formel III

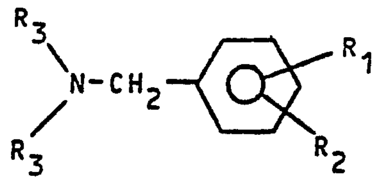

III

wobei $R_3$ $C_1$-$C_4$-Alkyl und $R_1$ Fluor und $R_2$ Wasserstoff oder Chlor bedeutet in Gegenwart von Alkalialkoholat versetzt wird.

3. Pharmazeutische Präparate, enthaltend als Wirkstoff eine Verbindung der Formel I beziehungsweise ein physiologisch verträgliches Säureadditionssalz davon in Kombination mit üblichen Hilfs-und/oder Trägerstoffen.

4. Verfahren zur Herstellung von pharmazeutischen Präparaten nach Anspruch 3, dadurch gekennzeichnet, daß man eine Verbindung der Formel I beziehungsweise deren physiologisch verträglichen Säureadditionssalze mit üblichen galenischen Hilfs-und/oder Trägerstoffen zu üblichen pharmazeutischen Anwendungsformen verarbeitet.

5. Verwendung einer Verbindung der Formel I und deren physiologisch verträglichen Säureadditionssalze zur Behandlung von Schmerzen und Fieberzuständen des menschlichen Organismus.

6. Verwendung einer Verbindung der Formel I und deren physiologisch verträglichen Säureadditionssalze zur Herstellung eines Arzneimittels zur Behandlung von Schmerz-und Fieberzuständen des menschlichen Organismus.

Europäisches Patentamt

# EUROPÄISCHER RECHERCHENBERICHT

Nummer der Anmeldung

EP 87 11 6036

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.3) |
|---|---|---|---|
| A | CHEMICAL ABSTRACTS, Band 97, Nr. 11, 13. Septembe 1982, Seite 753, Spalte 2, Zusammenfassungsnr. 92096q, Columbus, Ohio, US; W. CZUBA et al.: "Direct ring benzylaton of 2,6-diaminopyridine"; & POL. J. CHEM. 1981, 55(4), 931-934 | 1,2 | C 07 D 213/73<br>A 61 K 31/44 |
| A | CHEMICAL ABSTRACTS, Band 92, Nr. 19, 12. Mai 1980, Seite 588, Spalte 2, Zusammenfassungsnr. 163817k, Columbus, Ohio, US; B.S. RAUCKMAN et al.: "2,4-Diamino-5-benzylpyrimidines and analogs as antibacterial agents. 3. C-Benzylation of aminopyridines with phenolic Mannich bases. Synthesis of 1- and 3-deaza analogs of trimethoprim"; & J. MED. CHEM. 1980, 23(4), 384-391 | 1,2 | |
| A | CHEMICAL ABSTRACTS, Band 93, Nr. 3, 21. Juli 1980, Seite 586, Spalte 1, Zusammenfassungsnr. 24993x, Columbus, Ohio, US; & JP - B - 55 000 028 (AJINIMOTO CO. INC.) 05.01.1980 | | RECHERCHIERTE SACHGEBIETE (Int. Cl.3)<br>C 07 D 213/00<br>A 61 K 31/00 |

Der vorliegende Recherchenbericht wurde für alle Patentansprüche erstellt

| Recherchenort | Abschlußdatum der Recherche | Prüfer |
|---|---|---|
| BERLIN | 12-01-1988 | VAN AMSTERDAM L.J.P. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)